# EUROPÄISCHE PATENTSCHRIFT

(11) **EP 0 720 551 B1**
(45) Veröffentlichungstag und Bekanntmachung des Hinweises auf die Patenterteilung: **27.05.1998**
(21) Anmeldenummer: 94926804.9
(22) Anmeldetag: 21.09.1994
(51) Int. Cl.: B60T 8/36, F16K 27/02

(54) **HYDRAULIKAGGREGAT FÜR SCHLUPFGEREGELTE BREMSANLAGEN VON KRAFTFAHRZEUGEN**
HYDRAULIC SYSTEM FOR ANTI-LOCK BRAKING INSTALLATIONS IN MOTOR VEHICLES
APPAREIL HYDRAULIQUE POUR SYSTEMES DE FREINAGE ANTIPATINAGE D'AUTOMOBILES

(30) Priorität: 24.09.1993 DE 4332538
(43) Veröffentlichungstag der Anmeldung: 10.07.1996
(73) Patentinhaber: ROBERT BOSCH GMBH, 70442 Stuttgart (DE)
(72) Erfinder: FRIEDOW, Michael, D-71732 Tamm (DE); LANDER, Jürgen, D-70563 Stuttgart (DE); STAIB, Helmut, D-71701 Schwieberdingen (DE); MÜLLER, Klaus, D-71732 Tamm (DE)
(86) Internationale Anmeldenummer: DE9401098
(87) Internationale Veröffentlichungsnummer: WO9508462

(56) Entgegenhaltungen:
- WO-A-91/17378
- WO-A-92/12878
- DE-A- 3 810 581
- DE-A- 4 030 571
- DE-A- 4 142 004
- DE-A- 4 330 616
- US-A- 4 312 380
- PATENT ABSTRACTS OF JAPAN vol. 16, no. 438 (M-1309) 11. September 1992 & JP,A,04 151 080 (NISSHINBO IND.) 25. Mai 1992

## Beschreibung

### Stand der Technik

Die Erfindung geht aus von einem Hydraulikaggregat für schlupfgeregelte Bremsanlagen von Kraftfahrzeugen nach der Gattung des Hauptanspruchs.

Durch die Druckschrift DE 38 10 581 A1 ist es schon bekannt, ein solches Hydraulikaggregat mit einem Ventilblock aus Stahl herzustellen, in diesen abgestufte Aufnahmebohrungen für den hydraulischen Teil elektromagnetisch betätigter Ventile einzubringen, den Ventilen Befestigungsflansche zuzuordnen, diese in die Stufenbohrungen einzuführen und zur druckdichten Lagesicherung der Ventile den Werkstoff Stahl des Ventilblocks gegen die Befestigungsflansche zu stemmen. In der Druckschrift DE 38 10 581 A1 ist ausdrücklich darauf hingewiesen, daß es der Vorgang des Verstemmens unabdingbar mache, nur Stahl als Werkstoff heranzuziehen. Dadurch seien derartige Hydraulikaggregate schwer, das Verstemmen teuer und nicht ohne Probleme, insbesondere im Hinblick auf die Dichtung. Statt dessen wird dort für eine Leichtbauweise aus Aluminium vorgeschlagen, mindestens ein Elektromagnetventil zwischen zwei aus Aluminium bestehende Platten mittels Zugschrauben einzuspannen. Beim in der Druckschrift DE 38 10 581 A1 als nachteilig offenbarten Entwicklungsstand ist der Befestigungsflansch des jeweiligen Elektromagnetventils am offenen Ende eines kapselförmigen Ventildomes, der magnetisch wirksame Elemente, wie Anker und Magnetkern, enthält, durch trichterförmiges Aufweiten hergestellt. Entsprechend der Form des Befestigungsflansches ist die Gegenfläche der Bohrungsstufe der Aufnahmebohrung in kostenaufwendiger Weise gestaltet. Der durch das Verstemmen verdrängte Werkstoff des Ventilblocks umschließt den Befestigungsflansch in vollem Umfang. Dennoch genügt diese Prägeverbindung offenbar nicht den Festigkeitsanforderungen, denn in der Druckschrift DE 40 30 571 A1 ist beschrieben, in die Aufnahmebohrung des Ventilblocks eine an die Form des Befestigungsflansches angepaßte Buchse einzulassen und neben der durch die Prägeverbindung erzielten reibschlüssigen Einspannung des Flansches zusätzlich eine formschlüssige Verbindung vorzusehen, um der zwischen den Kontaktflächen der erwähnten Elemente wirkenden Schubkraft entgegenzuwirken. Dies erhöht jedoch den Kostenaufwand für die Befestigung des hydraulischen Teils des Elektromagnetventils im Ventilblock.

Ferner ist aus WO 92/12878 A1 ein Hydraulikaggregat mit einem Ventilblock aus Aluminium bekannt, in dem der hydraulische Teil elektromagnetisch betätigter Ventile aufgenommen ist. Über die Art der Befestigung des hydraulischen Teils der Ventile ist keine Angabe gemacht.

Bei einem durch die gattungsbildende Druckschrift DE 41 42 004 A1 bekannten elektromagnetisch betätigten Ventil für Bremsanlagen der eingangs genannten Art hat dessen Ventildom einen radial abstehenden Befestigungsflansch, der mittels einer über den Ventildom geschobenen Schraubhülse in einem Ventilblock abgedichtet gehalten ist. Das Befestigen mittels der Schraubhülse und eines im Ventilblock für diese angeordneten Einschraubgewindes ist teuer. Außerdem sind solche Einschraubgewinde schwieriger zu reinigen als glatte Durchmesserstufen von Bohrungen. Sorgfältige Reinigung ist aber unabdingbar, weil nicht entfernte Späne zu Undichtheit zwischen dem Befestigungsflansch und dem Ventilblock führen oder in den Hydraulikkreis gelangen können, was schwerwiegende Schäden oder Funktionsverlust des Hydraulikaggregats hervorrufen kann.

### Vorteile der Erfindung

Das erfindungsgemäße Hydraulikaggregat mit dem kennzeichnenden Merkmal des Hauptanspruchs hat demgegenüber den Vorteil, daß neben der preisgünstig herstellbaren Verbindung des wenigstens einen Ventils mit dem Ventilblock auch eine erhebliche Gewichtsersparnis durch die Wahl des gegenüber Stahl wesentlich leichteren Werkstoffs erreicht ist.

Durch die in den Unteransprüchen aufgeführten Maßnahmen sind vorteilhafte Weiterbildungen und Verbesserungen des im Hauptanspruch angegebenen Hydraulikaggregats möglich.

Mit der im Anspruch 2 gekennzeichneten Ausgestaltung wird einerseits auf einfache Weise eine Ableitung von auf den hydraulischen Teil des Ventils einwirkenden Kräften auf den Ventilblock erzielt, andererseits wird durch die Formgebung der Buchse beim an sich beliebig durchführbaren Verstemmen des Befestigungsflansches eine Verformung des Ventildomes vermieden und die freie Beweglichkeit der innerhalb des Ventildomes angeordneten Elemente unterstützt.

Die im Anspruch 3 angegebene Weiterbildung zeichnet sich neben einer wenig aufwendigen Herstellung von Aufnahmebohrung und Befestigungsflansch auch durch eine überwiegend als Formschluß ausgestaltete Verbindung zwischen dem hydraulischen Teil des Ventils und dem Ventilblock aus, die hohe Belastungen aufnehmen kann.

Mit der Verbesserung nach Anspruch 4 wird aufgrund des Buchsenwerkstoffs auf der einen Seite eine hohe Gestaltfestigkeit der Buchse beim Verstemmen ihres Befestigungsflansches und auf der anderen Seite eine günstige Leitung des Magnetflusses zwischen dem Gehäuse des elektrischen Teils und den magnetisch wirksamen Elementen des hydraulischen Teils des Ventils erzielt. Die Buchse hat somit einen Doppelnutzen.

### Zeichnung

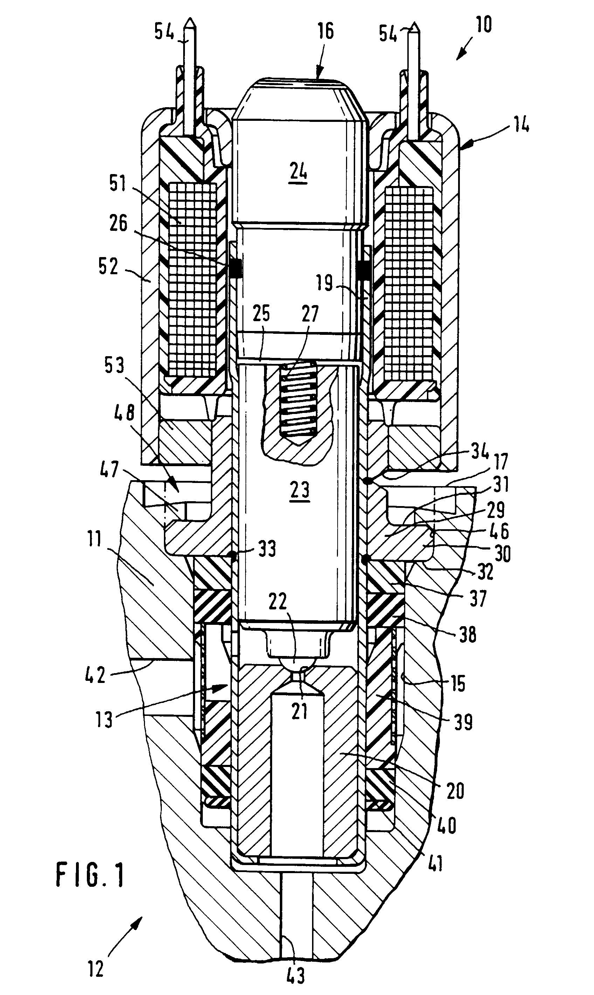

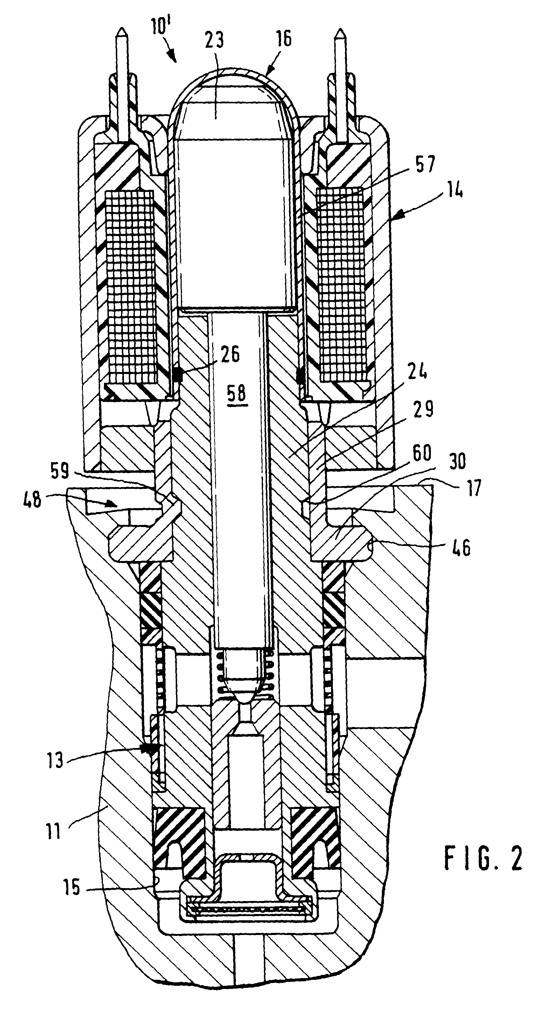

Zwei Ausführungsbeispiele der Erfindung sind in der Zeichnung vereinfacht dargestellt und in der nachfolgenden Beschreibung näher erläutert. Es zeigen Figur 1 einen Längsschnitt durch ein stromlos geschlossenes, elektromagnetisch betätigtes Ventil, welches durch eine Prägeverbindung mit einem Ventilblock eines Hydraulikaggregats verbunden ist, und Figur 2 eine entsprechende Darstellung eines stromlos offenen Ventils.

### Beschreibung der Ausführungsbeispiele

Ein in Figur 1 als erstes Ausführungsbeispiel dargestelltes, elektromagnetisch betätigtes Ventil 10 ist an einem Ventilblock 11 angeordnet und bildet einen Teil eines im übrigen nicht gezeichneten Hydraulikaggregats 12 für schlupfgeregelte Bremsanlagen von Kraftfahrzeugen. Das Ventil 10 besteht aus einem hydraulischen Teil 13 und einem elektrischen Teil 14. Der hydraulische Teil 13 ist im wesentlichen in einer gestuften Aufnahmebohrung 15 des aus einer duktilen Aluminium-Legierung bestehenden Ventilblocks 11 aufgenommen und befestigt. In Verlängerung der Aufnahmebohrung 15 überragt der hydraulische Teil 13 mit einem Ventildom 16 eine Begrenzungsebene 17 des Ventilblocks 11. Auf den Ventildom 16 ist der elektrische Teil 14 aufgesteckt.

Der hydraulische Teil 13 weist eine dünnwandige Führungshülse 19 kreiszylindrischen Querschnitts auf. Von der Aufnahmebohrung 15 ausgehend ist in der Führungshülse 19 ein Ventilkörper 20 mit Preßsitz aufgenommen. Der Ventilkörper 20 hat einen Ventilsitz 21 für ein Schließglied 22 eines in der Führungshülse 19 längsbewegbaren Ankers 23. Am vom Ventilkörper 20 abgewandten Ende ist die Führungshülse 19 durch einen Magnetkern 24 als Teil des Ventildoms 16 verschlossen. Der Magnetkern 24 greift unter Belassen eines Luftspalts 25 zum Anker 23 mit Preßsitz in die Führungshülse 19 ein und ist mit dieser durch eine umlaufende Schweißung 26 verbunden. Diese Verbindung ist druckdicht und hydraulisch hoch belastbar. Im Anker 23 ist eine am Magnetkern 24 angreifende Schließfeder 27 aufgenommen, welche in der gezeichneten Ruhestellung des Ventils 10 das Schließglied 22 am Ventilsitz 21 anliegend hält: Das Ventil 10 ist somit stromlos geschlossen.

Auf den mittleren Bereich der Führungshülse 19, und zwar radial außerhalb des magnetisch wirksamen Ankers 23, ist eine Buchse 29 aufgeschoben. Die Buchse 29 hat eine größere Wanddicke als die Führungshülse 19 des Ventildoms 16. Gegen den Ventilkörper 20 ist die Buchse 29 mit einem radial abstehenden Befestigungsflansch 30 versehen, dessen beide Stirnflächen 31 und 32 rechtwinklig zur Achse der Aufnahmebohrung 15, welche zugleich die Längsachse des Ventils 10 ist, verlaufen. Die Buchse 29 besteht aus magnetisierbarem Werkstoff, beispielsweise weichmagnetischem Stahl. Sie ist mit der aus nicht rostendem Stahl bestehenden Führungshülse 19 durch Schweißung 33 fest verbunden. Diese kann, wie dargestellt, stirnseitig der Buchse 29 erfolgen; sie kann auch im Bereich einer oder mehrerer Ausnehmungen 34 liegen, mit denen die Wanddicke der Buchse 29 im Bereich der Begrenzungsebene 17 vermindert ist. Die Schweißung 33 kann den vollen Umfang der Führungshülse 19 oder nur Teile davon erfassen. Anstelle der Schweißung 33 kann die Verbindung der Buchse 29 mit der Führungshülse 19 auch durch Klebung erfolgen.

Vom Befestigungsflansch 30 ausgehend sind auf den der Aufnahmebohrung 15 zugeordneten Teil der Führungshülse 19 ein Stützring 37 und ein Dichtring 38 aufgenommen, der die Aufnahmebohrung 15 nach außen dicht verschließt. Auf den Dichtring 38 folgen eine Filterhülse 39, ein zweiter Dichtring 40 sowie ein Stützring 41. Der zweite Dichtring 40 trennt Druckmittelleitungen 42 und 43 des Ventilblocks 11, deren Durchgang mit dem Ventil 10 schaltbar ist.

Der formsteif ausgebildete Befestigungsflansch 30 der Buchse 29 ist in einer Bohrungsstufe 46 der Aufnahmebohrung 15 aufgenommen. Die ursprüngliche Kontur der Bohrungsstufe 46 ist mit strichpunktierten Linien angedeutet: Der Durchmesser der Bohrungsstufe ist somit kleiner als der Durchmesser des elektrischen Teils 14 des Ventils 10. Die ventilkörperseitige Stirnfläche 32 des Befestigungsflansches 30 liegt am Grund der Bohrungsstufe 46 an. Die andere Stirnfläche 31 des Befestigungsflansches 30 ist dagegen durch einen Werkstoffwulst 47 überdeckt, der durch eine Verstemmung 48 des vom Bohrungsrand verdrängten Werkstoffs erzielt ist. Der von dieser Prägeverbindung beaufschlagte Bereich am Bohrungsrand der Stufe 46 liegt ebenfalls innerhalb des Durchmessers des elektrischen Teils 14. Der Werkstoffwulst 47 erfaßt den Befestigungsflansch 30 in vollem Umfang und sichert die Lage des hydraulischen Teils 13 im Ventilblock 11. Er vermag die innerhalb und außerhalb des hydraulischen Teils 13 wirksam werdenden und auf den Befestigungsflansch 30 übertragenen Kräfte sicher in den Ventilblock 11 abzuleiten. Bei geringerer Beanspruchung kann es ausreichend sein, wenn nur Abschnitte des Befestigungsflansches 30 von Teilen des Werkstoffwulstes 47 erfaßt sind.

Der elektrische Teil 14 des Ventils 10 ist nach dem Befestigen des hydraulischen Teils 13 im Ventilblock 11 auf den Ventildom 16 im Bereich der magnetisch wirksamen Elemente Anker 33 und Magnetkern 24 aufgesteckt. Der elektrische Teil 14 hat eine elektrische Spule 51, welche den Ventildom 16 im Bereich des Magnetkerns 24 umschließt. Die Spule 51 ist von einem Gehäuse 52 aus weichmagnetischem Werkstoff übergriffen, in das bodenseitig eine gleichfalls aus weichmagnetischem Werkstoff bestehende Ringscheibe 53 eingepreßt ist. Auf der von der Begrenzungsebene 17 abgewandten Stirnseite des Gehäuses 52 sind Anschlußstifte 54 der Spule 51 ausgebildet. Das Gehäuse 52 des elektrischen Teils 14 umgreift vorzugsweise spielfrei einerseits den Magnetkern 24 und andererseits mit seiner Ringscheibe 53 die Buchse 29 des hydraulischen Teils 13 an. Bei erregter elektrische Spule 51 trägt die Buchse 29 ebenso wie der Magnetkern 24, das Gehäuse 52 und die Ringscheibe 53 zum Leiten des Magnetflusses auf den Anker 23 des hydraulischen Teils 13 bei. Der magnetisch wirksame Magnetkern 24 überführt den Anker 23 in die Offenstellung des Ventils 10.

Beim in Figur 2 dargestellten zweiten Ausführungsbeispiel besitzt das elektromagnetisch betätigte Ventil 10' ebenfalls einen hydraulischen Teil 13 und einen elektrischen Teil 14. Das Ventil 10 ist von der Bauform her in seiner Ruhestellung stromlos offen geschaltet. Es hat einen Ventildom 16 mit einer dünnwandigen Kapsel 57, in welcher der Anker 23 des Ventils 10' längsbewegbar aufgenommen ist. Die Kapsel 57 ist analog zum ersten Ausführungsbeispiel mit dem bis in die gestufte Aufnahmebohrung 15 verlängerten Magnetkern 24 durch Schweißung 26 verbunden. Der von einem Ventilstößel 58 durchdrungene Magnetkern 24 besitzt eine relativ große Wandstärke. Im Bereich der Begrenzungsebene 17 des Ventilblocks 11 ist auf den magnetisch wirksamen Magnetkern 24 die Buchse 29 mit ihrem in der Bohrungsstufe 46 aufgenommenen Befestigungsflansch 30 geschoben. Die Verbindung der Buchse 29 mit dem Magnetkern 24 ist durch eine Prägeverbindung erzielt, aufgrund der Einprägungen 59 der Buchse in eine Nut 60 oder in Ausnehmungen des Magnetkerns 24 eingreifen. Der Befestigungsflansch 30 des hydraulischen Teils 13 ist wie beim ersten Ausführungsbeispiel durch eine Verstemmung 48 mit dem Ventilblock 11 verbunden. Ebenfalls in gleicher Weise ist der elektrische Teil 14 auf den Ventildom 16 des Ventils 10 aufgesteckt.

Bei einer Mehrfachanordnung von Ventilen 10 beziehungsweise 10' im Verlauf der Begrenzungsebene 17 des Ventilblocks 11 ist eine hohe Packungsdichte erreichbar, da die Verstemmung 48 innerhalb des Durchmessers des elektrischen Teils 14 liegt. Ventile 10 beziehungsweise 10' können daher mit sehr geringem Abstand nebeneinander angeordnet werden.

## Patentansprüche

1. Hydraulikaggregat (12) für schlupfgeregelte Bremsanlagen von Kraftfahrzeugen,
mit einem Ventilblock (11) aus Metall mit wenigstens einer gestuften Aufnahmebohrung (15) für den hydraulischen Teil (13) eines elektromagnetisch betätigten Ventils (10),
mit einem am hydraulischen Teil (13) angeordneten Befestigungsflansch (30), der in eine Stufe (46) der Aufnahmebohrung (15) eingesetzt und am Ventilblock (11) befestigt ist,
mit einem magnetisch wirksame Elemente, wie Anker (23) und Magnetkern (24) des hydraulischen Teils (13) enthaltenden druckdichten Ventildom (16), der in Verlängerung der Aufnahmebohrung (15) über eine Begrenzungsebene (17) des Ventilblocks (11) greift,
und mit einem elektrischen Teil (14) des Ventils (10), welcher auf den Ventildom (16) aufgesteckt ist und eine diesen umschließende, elektrische Spule (51) sowie ein magnetflußleitendes Gehäuse (52) aufweist,
dadurch gekennzeichnet,
daß der Werkstoff des Ventilblocks (11) ein Leichtmetall, wie Aluminium-Legierung oder dergleichen, ist,
daß der Befestigungsflansch (30) des hydraulischen Teils (13) des Ventils (10) durch eine Verstemmung (48) aus dem Metall des Ventilblocks (11) lagegesichert ist und daß im Gehäuse (52) auf der der Begrenzungsebene (17) zugewandten Seite eine Ringscheibe (53) aus einem weichmagnetischen Werkstoff zum Leiten des Magnetflusses aufgenommen ist.

2. Hydraulikaggregat nach Anspruch 1, dadurch gekennzeichnet, daß der Befestigungsflansch (30) radial von einer mit dem Ventildom (16) fest verbundenen Buchse (29) absteht, deren Wanddicke größer ist als die einer die magnetisch wirksamen Elemente des Ventildoms (16) aufnehmenden Hülse.

3. Hydraulikaggregat nach Anspruch 2, dadurch gekennzeichnet, daß der Befestigungsflansch (30) rechtwinklig zur Achse der Aufnahmebohrung (15) verlaufende Stirnflächen (31, 32) hat, von denen die eine (32) an der Bohrungsstufe (46) anliegt und die andere (31) durch vom Bohrungsrand verdrängten Werkstoff (47) wenigstens teilweise überdeckt ist.

4. Hydraulikaggregat nach Anspruch 2, dadurch gekennzeichnet, daß die Buchse (29) aus einem magnetisierbaren Werkstoff besteht, radial außerhalb eines der magnetisch wirksamen Elemente, wie Anker (23) und Magnetkern (24) des hydraulischen Teils (13) angeordnet und umfangsseitig vom Magnetfluß leitenden Gehäuse (52) des elektrischen Teils (14) des Ventils (10) wenigstens mittelbar umgriffen ist.

## Claims

1. Hydraulic assembly (12) for anti-lock brake systems of motor vehicles, with a valve block (11) made of metal, having at least one stepped receiving bore (15) for the hydraulic part (13) of an electromagnetically actuated valve (10), with a fastening flange (30) which is arranged on the hydraulic part (13) and which is inserted into a step (46) of the receiving bore (15) and is fastened to the valve block (11), with a pressure-tight valve dome (16) which contains magnetically active elements, such as an armature (23) and magnet core (24) of the hydraulic part (13) and which reaches, in the extension of the receiving bore (15), beyond a limiting plane (17) of the valve block (11), and with an electrical part (14) of the valve (10), the said part being slipped onto the valve dome (16) and having an electrical coil (51), surrounding the latter, and a housing (52) which conducts the magnetic flux, characterized in that the material of the valve block (11) is a light metal, such as an aluminium alloy or the like, in that the fastening flange (30) of the hydraulic part (13) of the valve (10) is secured in position by means of caulking (48) made from the metal of the valve block (11), and in that an annular disc (53) made from a magnetically soft material for conducting the magnetic flux is received in the housing (52) on the side facing the limiting plane (17).

2. Hydraulic assembly according to Claim 1, characterized in that the fastening flange (30) projects radially from a bush (29) which is connected fixedly to the valve dome (16) and the wall thickness of which is greater than that of a sleeve receiving the magnetically active elements of the valve dome (16).

3. Hydraulic assembly according to Claim 2, characterized in that the fastening flange (30) has end faces (31, 32) which run at right angles to the axis of the receiving bore (15) and of which one (32) bears on the bore step (46) and the other (31) is at least partially covered by material (47) displaced from the bore edge.

4. Hydraulic assembly according to Claim 2, characterized in that the bush (29) consists of a magnetizable material, is arranged radially outside one of the magnetically active elements, such as the armature (23) and magnet core (24), of the hydraulic part (13) and is at least indirectly surrounded circumferentially by the housing (52) of the electrical parts (14) of the valve (10), the said housing conducting the magnetic flux.

## Revendications

1. Ensemble hydraulique (12) pour des installations de frein de véhicules automobiles à régulation de patinage, comprenant :
- un bloc soupape (11) en métal, ayant au moins un perçage de réception (15), étagé, pour la partie hydraulique (13) d'une soupape électromagnétique (10),
- une bride de fixation (30) prévue sur la partie hydraulique (13), cette bride fixée au bloc-soupape (11), étant logée dans une partie étagée (46) du perçage de réception (15),
- un dôme de soupape (16) comportant, de manière étanche à la pression, les éléments à effet magnétique tels que l'induit (23) et le noyau magnétique (24) de la partie hydraulique (13), ce dôme venant dans le prolongement du perçage de réception (15), au-delà du plan de limite (17) du bloc à soupape (11),
- et une partie électrique (14) de la soupape (10) emmanchée sur le dôme de soupape (16) et comportant une bobine électrique (51) entourant ce dôme ainsi qu'un boîtier (52) conducteur de flux magnétique,
caractérisé en ce que
la matière du bloc à soupape (11) est un métal léger tel qu'un alliage d'aluminium ou analogue,
- la bride de fixation (30) de la partie hydraulique (13) de la soupape (10 est bloquée en position par le matage (48) du métal du bloc-soupape (11) et
- le boîtier (52), du côté tourné vers le plan limite (17) comporte une rondelle annulaire (53) en matière à aimantation douce pour conduire le flux magnétique.

2. Ensemble hydraulique selon la revendication 1,
caractérisé en ce que
la bride de fixation (30) est radialement en saillie d'un manchon (29) relié solidairement au dôme de soupape (16), manchon dont la paroi est plus épaisse que celle du manchon recevant les éléments à action magnétique du dôme de soupape (16).

3. Ensemble hydraulique selon la revendication 2,
caractérisé en ce que
la bride de fixation (30) présente des surfaces frontales (31, 32) perpendiculaires à l'axe du perçage de réception (15), surfaces dont l'une (32) s'applique contre la partie étagée (46) du perçage et l'autre (31) est recouverte au moins partiellement par la matière (47) refoulée, du bord du perçage.

4. Ensemble hydraulique selon la revendication 2,
caractérisé en ce que
le manchon (29) en matière aimantée, est prévu radialement à l'extérieur de l'un des éléments à effet magnétique tel que l'induit (23) et le noyau magnétique (24) de la partie hydraulique (13) et il est entouré du côté périphérique au moins indirectement par le boîtier conducteur de flux (52) de la partie électrique (14) de la soupape.
